# EUROPEAN PATENT SPECIFICATION

(11) **EP 0 374 315 B1**
(45) Date of publication and mention of the grant of the patent: **28.07.1993**
(21) Application number: 88312083.4
(22) Date of filing: 20.12.1988
(51) Int. Cl.: B01J 27/232, B01J 23/04, C07C 2/72

(54) **Catalyst system, process for preparing it, and process for alkylating toluene**
Katalytisches System, Verfahren zu seiner Herstellung und Verfahren für die Alkylierung von Toluen
Système catalytique, procédé pour le préparer et procédé d'alkylation du toluène

(43) Date of publication of application: 27.06.1990
(73) Proprietor: NESTE OY, FIN-02150 Espoo (FI)
(72) Inventor: Pekka, Knuuttila, F-6499 Porvoo (FI); Aila, Ali-Hokka, F-06850 Kulloo (FI)
(74) Representative: Lamb, John Baxter

(56) References cited:
- WO-A-88/04955
- GB-A- 1 269 280

## Description

The present invention concerns a catalyst system for use in the alkylation of toluene, a process for preparing the catalyst, and a process for alkylating toluene.

Alkali metal catalysts are, as is known in the art, well suited for side-chain alkylation of alkyl aromatics with olefins; the catalyst having metallic sodiumor potassium dispersed onto the surface of an inorganic or graphite carrier. For instance, in Finnish patent application No. 865362 there is disclosed a catalyst system containing metallic sodium on a K₂CO₃ carrier. In GB-A-1,269,280 there is described a catalyst which is produced by dispersing sodium and/or lithium onto a non-aqueous potassium compound. In addition, reference may be made to Finnish patent application No. 865363 which discloses a catalyst system which contains metallic sodium dispersed thermally from a sodium-containing compound onto the surface of solid K₂CO₃. WO 88/04955 discloses a catalyst system comprising metallic sodium on a K₂CO₃ carrier together with, as an esterified component, a transition metal oxide.

Drawbacks of the said catalysts are, however, the cumbersome manufacturing and processing of the catalysts, a relatively low activity at high temperatures, and a rapid decrease of activity as a consequence of the catalyst becoming tarred and coked.

The catalyst of the present invention represents improvement in comparison with the above-mentioned catalysts in that the starting materials are easier to process, and, yet, the catalyst is as active as the alkali metal catalyst.

The catalyst system of the present invention is characterised in that it contains sodium on a potassium carbonate (K₂CO₃) carrier, whereby at least a major part of the sodium is in the form of sodium oxide, and is prepared by mixing sodium oxide (Na₂O) and potassium carbonate (K₂CO₃) and heating the mixture at a temperature of 100-400°C, in the substantial absence of air.

The process for alkylating toluene is characterized in that the catalyst system is as defined above.

The production of the catalyst of the invention is carried at in a simple manner by mixing sodium oxide and potassium carbonate, and by heating the mixture. The heating takes place at 100 to 400° C temperature, but most advantageously and rapidly the heating takes place at 260 to 280°C temperature, whereby a preferable heating period is 0.5 to 1 hour. The heating is carried out advantageously, but not necessarily, at vacuum, so that it may be carried out in the substantial absence of air.

It is surprising that the catalyst of the invention should be active seeing that neither sodium oxide nor potassium carbonate alone displays any activity, and between potassium carbonate and sodium oxide, no chemical reactions take place, as evidenced by the fact that in the x-ray diffraction analysis of the completed catalysts, the intended phases, that is, sodium oxide and potassium carbonate, were identified.

The catalysts of the invention were tested with propylene by side-chain-alkylating the toluene.

The alkylating reaction can be illustrated with the following reaction equation.

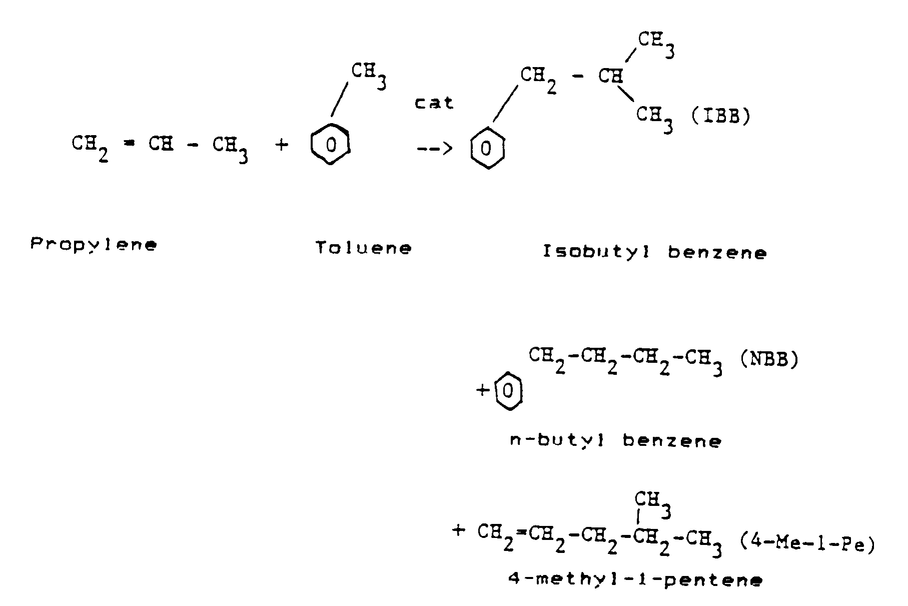

The main product produced in the reaction is isobutyl benzene; n-butyl benzene and a dimerization product of propylene, 4-methyl-1-pentene, is produced as byproduct as a result of by-reactions.

The tests were carried out in a 1 dm³ autoclave and in a continuous-action microreactor.

After the reaction, the gas and liquid phases were analyzed by gas-chromatography.

In the following, the invention is described in detail with the aid of a catalyst production example and an alkylating example. The invention is not, however, restricted to the details thereof.

### Catalyst production

The catalyst was produced in a 1 dm³ Parr steel reactor at 270°C temperature and at vacuum. The desired amount of sodium oxide and potassium carbonate were weighed into the reactor, which was closed, and vacuum was established. The mixture was heated 260 to 280°C, at which it was maintained for 0.5 to 1.0 h.

Six catalysts were prepared, and the sodium content varying from 0 to 100 per cent by weight.

### Analyzing catalysts

The completed catalysts were dark grey or brownish in colour. When producing catalysts containing sodium oxide of 14 to 90% by weight, a metallic phase could be observed on the walls of the reactor. Of the completed catalysts, a catalyst containing 40% by weight sodium oxide was analyzed using X-ray diffraction metric method (XRD). The phases identified in the diffraction analysis, present in the catalyst, were sodium oxide, potassium carbonate and probably metallic sodium. The sodium oxide had large crystals and its concentration was high. Potassium carbonate had either small crystals or it was coated by sodium oxide because the peaks were low in intensity and wide.

### Testing the catalysts

The catalysts were tested by side-chain alkylating the toluene with propylene. The tests were carried out in a 1 dm³ Parr autoclave and in a continuous-action microreactor.

### Tests in a charge reactor

For the reaction conditions were selected the following: reaction time t = 19 h, reaction temperature T = 175°C, toluene/propylene molar ratio n(T)n(P) = 0.7, catalyst mass m = 23.0 g.

The catalyst was loaded into the reactor under nitrogen atmosphere. The reactor was closed and vacuum was produced. Toluene was conducted into the reactor with the aid of the vacuum prevailing in the reactor through a valve in the reactor cover. Propylene was supplied in liquid form into the reactor.

After the reaction (175°C, 19 h), the gas and liquid phases were analyzed by gas-chromatography.

### Results of charge experiments

For the main product in the reaction was obtained isobutyl benzene (IBB). As the result of the by-reactions, the product mixture contained also n-butyl benzene (NBB), 4-methyl-1-pentene (4M1P) as a dimerization product of the propylene, and isomerization products of the 4M1PO, and various hexene isomers.

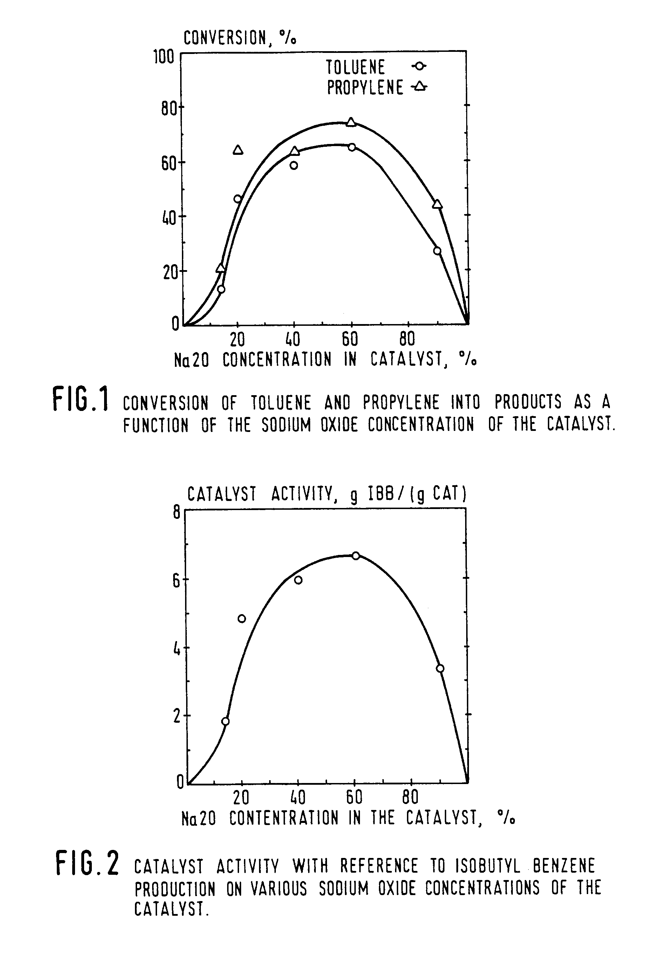

On Table 1 is presented the summary of the results of the test runs. From the composition of the product were calculated the conversions of the starting materials into products, the formation selectivities of various product components, and the activity of the catalyst regarding the IBB production. In Fig. 1 is presented the conversion of the toluene and propylene into products as a function of the sodium oxide concentration contained by the catalyst. In Fig. 2 is presented the activity of the catalyst in grams of IBB per catalyst gram (g IBB/ (g cat)).

**TABLE 1**

| Summary of the test runs with the Na₂O/K₂CO₃ catalyst. | | | | | | | | |
|---|---|---|---|---|---|---|---|---|
| CatConversions al. | | | Selectivities | | | | IBB/NBB ratio mol/mol | Cat.act. g IBB/g cat. |
| Na₂O % by w. | tol. % | prop. % | IBB % | NBB % | 4M1P % | ISOM. % | | |
| 0 | 0,0 | 0,0 | 0,0 | 0,0 | 0,0 | 0,0 | - | 0,0 |
| 14 | 13,4 | 20,7 | 68,4 | 6,2 | 8,4 | 4,0 | 11 | 1,8 |
| 20 | 46,2 | 64,0 | 66,4 | 7,4 | 4,8 | 8,0 | 9 | 4,8 |
| 40 | 58,4 | 63,5 | 71,7 | 7,5 | 4,7 | 8,7 | 10 | 6,0 |
| 60 | 64,8 | 73,8 | 70,9 | 7,1 | 3,5 | 9,1 | 10 | 6,6 |
| 90 | 26,5 | 43,7 | 72,8 | 8,3 | 6,6 | 7,7 | 9 | 3,3 |
| 100 | 0,0 | 0,0 | 0,0 | 0,0 | 0,0 | 0,0 | - | 0,0 |
| IBB = isobutyl benzene, NBB = n-butyl benzene, 4M1P = 4-methyl-1-pentene, ISOM = isomerization products of 4M1P | | | | | | | | |

On Table 1 and in Figs 1 and 2 are observed that the conversation as well as the activity of the starting materials are best with a catalyst in which the sodium concentration is 60% by weight. The result is not, however, significantly poorer with catalysts containing sodium oxide of 20 or 40% by weight.

When the Na₂O/K₂CO₃ catalyst is compared with a catalyst which is prepared from a pure alkali metal and alkaline metal carbonate (6% Na/K₂CO₃), the results are in comparing the conversions and the activity of the catalyst remarkably better with a 20, 40 and 60% oxide catalyst. With the Na/K₂CO₃ catalyst in tests carried out in the same conditions, the toluene conversion is 30% and the propylene conversion, 42% and the activity of the catalyst, 4 to 5 g IBB/ (g cat) when the conversions with catalysts containing 20 to 60% by weight sodium oxide are 46 to 65% and 64 to 74%, and the activity of the catalyst 5 to 7 g IBB/ (g cat).

In addition, it was observed that the oxide catalyst became readily activated, and no induction time was noted in the reaction. In other words, the pressure in the reactor started to fall immediately after the temperature had risen to 175°C. Instead, with the alkaline metal catalyst the starting of the reaction took about one hour.

A significant difference between the alkaline metal catalyst and the oxide catalyst is the high isomerization efficiency of the 4M1p in the latter. The formation selectivity of the isomerization products of the 4M1P with the oxide catalyst is about doubled compared with the alkaline metal catalyst. The isomerization may, however, be reduced by lowering the reaction temperature and shortening the reaction time.

### Continuous-action reactor

A 40% Na₂O/K₂CO₃ catalyst was selected for a continuous-action microreactor run. The parameters of the run were as follows: toluene feed about 9 g/h, propylene feed about 20 g/h, reactor temperature 170°C and pressure 90 bar. 26 g of the catalyst were loaded.

The Na₂O/K₂CO₃ catalyst appeared to be particularly suitable for producing isobutyl benzene in a continuous-action reactor because its tar formation (products heavier than NBB) is particularly low. The proportion of tars is not increased, either, with temperature increase and catalyst ageing. Although the Na₂O/K₂CO₃ catalyst is rather finely powdered, no pressure losses can be observed in the reactor because the tarring of the catalyst is non-existent. Therefore, the temperature of the reactor can be raised up tp 200°C. A drawback in the Na₂O/K₂CO₃ catalyst is its tendency to produce 4-methyl-2-pentene as a main dimerization product. Said isomerization tendency is strongly dependent on the temperature. The lowering of the temperature to 150°C returns the isomerization close to the isomerization ratio characteristic of the Na₂/K₂CO₃ catalyst.

**Table 3**

| Microreactor run with 40% Na₂O/K₂CO₃ catalyst | | | | | | | |
|---|---|---|---|---|---|---|---|
| Run time (h) | Tol.conv. (%) | Selectivities (%) | | | | Production h/gcath) | |
| | | 4M1P | other hex. | IBB | NBB | 4M1P | IBB |
| 73 | 48,6 | 32,6 | 11,0 | 49,0 | 6,6 | 0,15 | 0,23 |
| 162 | 32,8 | 23,0 | 8,0 | 57,1 | 5,2 | 0,07 | 0,16 |
| 260 | 10,0 | 17,8 | 7,3 | 63,9 | 5,5 | 0,05 | 0,16 |

## Claims

1. A catalyst system for use in the alkylation of toluene containing sodium on a potassium carbonate (K₂CO₃) carrier, whereby at least a major part of the sodium is in the form of sodium oxide, characterized in that it has been manufactured by mixing sodium oxide (Na₂O) and potassium carbonate, and heating said mixture at a temperature between 100°C and 400°C, in the substantial absence of air.

2. A catalyst system according to claim 1, characterized in that its sodium oxide concentration is from 10 to 70%.

3. A catalyst system according to claim 2 characterized in that its sodium oxide concentration is from 40 to 60%.

4. A process for producing a catalyst system according to any preceding claim, characterised in that it comprises mixing sodium oxide (Na₂O) and potassium carbonate (K₂CO₃) and heating said mixture at a temperature between 100°C and 400°C, in the substantial absence of air.

5. A process for alkylating toluene with propylene in the presence of a catalyst, characterised in that the catalyst is a catalyst system as claimed in any of claims 1-3.

## Patentansprüche

1. Katalysatorsystem zur Verwendung bei der Alkylierung von Toluol, enthaltend Natrium auf einem Kaliumcarbonat (K₂CO₃)-Träger, wobei mindestens ein größerer Teil des Natriums als Natriumoxid vorliegt, dadurch gekennzeichnet, daß es durch Vermischen von Natriumoxid (Na₂O) und Kaliumcarbonat und Erhitzen dieses Gemisches bei einer Temperatur zwischen 100°C und 400°C im wesentlichen unter Luftausschluß hergestellt wurde.

2. Katalysatorsystem nach Anspruch 1, dadurch gekennzeichnet, daß seine Natriumoxidkonzentration 10 bis 70% beträgt.

3. Katalysatorsystem nach Anspruch 2, dadurch gekennzeichnet, daß seine Natriumoxidkonzentration 40 bis 60% beträgt.

4. Verfahren zur Herstellung eines Katalysatorsystems nach einem der vorhergehenden Ansprüche, dadurch gekennzeichnet, daß es das Vermischen von Natriumoxid (Na₂O) und Kaliumcarbonat (K₂CO₃) und das Erhitzen dieses Gemisches bei einer Temperatur zwischen 100°C und 400°C im wesentlichen unter Luftausschluß beinhaltet.

5. Verfahren zur Alkylierung von Toluol mit Propylen in Gegenwart eines Katalysators, dadurch gekennzeichnet, daß der Katalysator ein Katalysatorsystem nach einem der Ansprüche 1-3 ist.

## Revendications

1. Un système catalytique utile dans l'alkylation du toluène, contenant du sodium sur un support de carbonate de potassium (K₂CO₃), dans lequel au moins une majeure partie du sodium est sous forme d'oxyde de sodium, caractérisé en ce qu'on l'a préparé par mélange d'oxyde de sodium (Na₂O) et de carbonate de potassium et chauffage dudit mélange à une température entre 100°C et 400°C, essentiellement en l'absence d'air.

2. Un système catalytique selon la revendication 1, caractérisé en ce que sa concentration en oxyde de sodium est de 10 à 70 %.

3. Un système catalytique selon la revendication 2, caractérisé en ce que sa concentration en oxyde de sodium est de 40 à 60 %.

4. Un procédé pour produire un système catalytique selon l'une quelconque des revendications précédentes, caractérisé en ce qu'il comprend le mélange d'oxyde de sodium (Na₂O) et de carbonate de potassium (K₂CO₃) et le chauffage dudit mélange à une température entre 100°C et 400°C, essentiellement en l'absence d'air.

5. Un procédé d'alkylation du toluène avec du propylène en présence d'un catalyseur, caractérisé en ce que le catalyseur est un système catalytique selon l'une quelconque des revendications 1 à 3.
